# EUROPÄISCHE PATENTSCHRIFT

(11) **EP 0 693 471 B1**
(45) Veröffentlichungstag und Bekanntmachung des Hinweises auf die Patenterteilung: **21.01.1998**
(21) Anmeldenummer: 95110635.0
(22) Anmeldetag: 07.07.1995
(51) Int. Cl.: C07C 49/683, C07C 49/753, C07C 49/747, C07C 45/74, A61K 7/42, A61K 31/12

(54) **Benzyliden-Norcampher-Derivate**
Benzylidene-norcamphor derivatives
Dérivés du benzylidène norcamphre

(30) Priorität: 23.07.1994 DE 4426216
(43) Veröffentlichungstag der Anmeldung: 24.01.1996
(73) Patentinhaber: MERCK PATENT GmbH, 64293 Darmstadt (DE)
(72) Erfinder: Stein, Inge, Dr., D-63110 Rodgau (DE); Schwarz, Michael, Dr., D-64521 Gross-Gerau (DE); Heywang, Ulrich, Dr., D-64289 Darmstadt (DE); Kompter, Michael, Dr., D-64560 Riedstadt (DE)

(56) Entgegenhaltungen:
- GB-A- 2 121 801
- TETRAHEDRON, , Bd. 48, Nr. 33, 1992 OXFORD GB, Seiten 6883-6896, W.R. BOWMAN ET AL. 'Radical Reactions of Bicyclo(2.2.1)heptan-3-spiro-2'-oxiranes'

## Beschreibung

Die Erfindung betrifft die Verwendung von Benzyliden-Norcampher-Derivaten der Formel I, 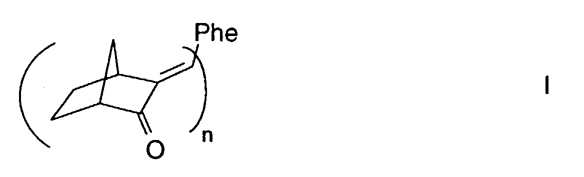 worin
- Phe: eine unsubstituierte oder durch 1 bis 5 Hydroxy-, Alkyl- oder Alkoxygruppen mit 1 bis 10 C-Atomen substituierte Phenyl-gruppe, und
- n: 1 oder 2
bedeuten,
als Lichtschutzfilter, sowie neue Verbindungen der Formel I, Verfahren zu ihrer Herstellung und ihre Verwendung in kosmetischen Zubereitungen, insbesondere zum Schutz vor Sonnenstrahlung, und in pharmazeutischen Zubereitungen zur vorbeugenden Behandlung von Entzündungen und Allergien der Haut oder bestimmter Krebsarten.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Es ist bekannt, daß die in kosmetischen Präparaten enthaltenen Komponenten nicht immer genügend lichtstabil sind und sich unter der Einwirkung von Lichtstrahlen zersetzen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbierten können, d.h. auch UV-B-Strahlen mit einer Wellenlänge zwischen 280 und 320 nm, die bei der Bildung eines Sonnenerythems eine entscheidende Rolle spielen, wie auch UV-A-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische Reaktionen auslösen können.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet.

Das Benzylidencampher-Derivat Eusolex® 6300 weist z.B. eine Extinktion von etwa 1 bei 305 nm auf.

Dibenzoylmethane wie Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut in vereinzelten Fällen begünstigen kann. Die ebenfalls als UVA-Filter verwendeten Benzophenone sind in den in der Kosmetik verwandten Ölen nur begrenzt löslich, und sie weisen eine relativ geringe Absorption auf. Dagegen sind nur wenige wasserlösliche UVA-Filter derzeit bekannt, deren UV-Absorption jedoch gering ist.

Ähnliche Biscampherylidenmethylphenylen sind z.B. aus der EP 0 390 682 bekannt; diese weisen jedoch vergleichsweise niedrige Extinktionswerte im UVA-Bereich auf.

W. Bowman et. al, Tetrahedron 48 (33), 6883-96, 1992, beschreiben unter anderem die Herstellung und Epoxidierung von folgenden Norcampher-Derivaten: 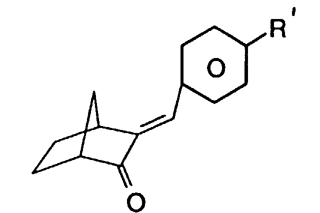 R' = H, Cl oder OCH₃

Es findet sich dort kein Hinweis, daß solche Verbindungen als Lichtschutzfilter eingesetzt werden können.

Es wurde gefunden, daß Norcampher-Derivate der Formel I, worin Phe eine unsubstituierte oder durch 1 bis 5 Hydroxy-, Alkyl- oder Alkoxygruppen substituierte Phenylgruppe bedeutet, insbesondere eine mono- oder unsubstituierte Phenylgruppe, hervorragende UVB-Filter-Eigenschaften besitzen (n = 1), bzw. hervorragende UVA-Filter-Eigenschaften besitzen (n = 2). Ihre Löslichkeit in den in der Kosmetik verwandten Ölen ist sehr gut, so daß Einsatzkonzentrationen auch in komplizierten Formulierungen bis mindestens 10 % der Zubereitung möglich sind.

Weiterhin weisen die erfindungsgemäßen Verbindungen eine außerordentliche Photostabilität gegenüber UV-Strahlung auf, die die Stabilität bisher bekannter UV-Filtersubstanzen bei weitem übersteigt, sie eignen sich insbesondere als UVB- oder UV-Breitbandfilter.

Falls die Extinktion im UVA-Bereich ein Minimum aufweist, ist das kein Nachteil, da man problemlos einen UVA-Filter mit in die Formulierung einarbeiten kann.

Weiterhin können die Verbindungen der Formel I auch zur vorbeugenden Behandlung von Entzündungen und Allergien der Haut und zur Verhütung bestimmter Krebsarten verwendet werden.

Außer ihren guten Eigenschaften als Filter zeichnen sich die erfindungsgemäßen Verbindungen durch eine gute thermische und photochemische Stabilität aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Fett-Trägern einen kontinuierlichen Film bilden; sie können auf diese Weise auf die Haut aufgetragen werden, um einen wirksamen Schutzfilm zu bilden.

Gegenstand der Erfindung ist somit die Verwendung der Verbindung der Formel I als Lichtschutzfilter, sowie die Verbindungen der Formel I, wobei folgende Verbindungen ausgenommen sind: 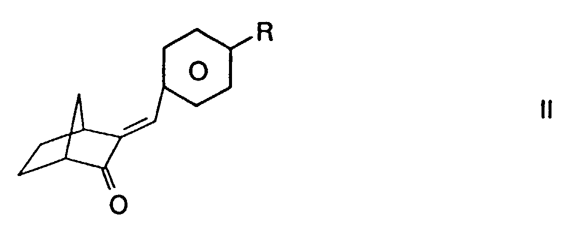 worin R H oder OCH₃
bedeuten,
insbesondere worin Phe durch 1 bis 2 Alkylgruppen mit 1 bis 2 C-Atomen substituiertes 1,4-Phenyl bedeutet.

Im Falle von n = 1 ist Phe eine unsubstituierte oder mit 1-5 Substituenten substituierte Phenylgruppe. Im Falle n = 2 ist Phe eine unsubstituierte oder mit 1-4 Substituenten substituierte Phenylengruppe.

Vorzugsweise ist Phe eine Phenyl-Gruppe der Formel (n = 1) 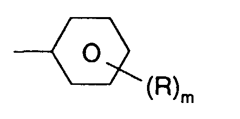 oder eine Phenylen-Gruppe der Formel (n = 2)

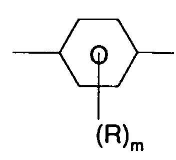

In dieser Formel steht R für einen Methyl-, Ethyl-, n-Propyl-, i-Propyl-, n-Butyl-, i-Butyl-, t-Butyl oder 1,1,3,3-Tetramethylbutylrest, Methoxy-, Ethoxy-, 2-Ethylhexyloxyrest oder Wasserstoff, vorzugsweise für Wasserstoff.

m bedeutet 1 bis 5, vorzugsweise 1 oder 2.

Vorzugsweise ist die Phenyl- bzw. Phenylengruppe unsubstituiert oder substituiert mit einer oder zwei Alkoxygruppen mit 1 bis 8 C-Atomen, insbesondere mit Methoxy, Ethoxy- oder 2-Ethylhexyloxygruppen.

Bevorzugte Verbindungen der Formel I sind diejenigen der Formeln I1 bis 115, wobei A eine Gruppe der Formel 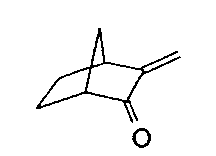 ist und
- R: Alkyl oder Alkoxy mit 1 bis 10 C-Atomen bedeutet.

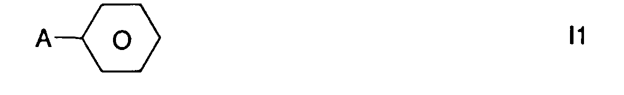

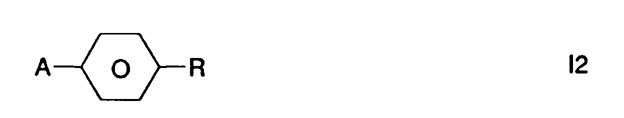

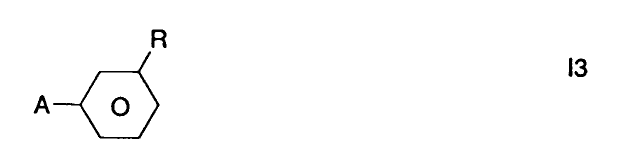

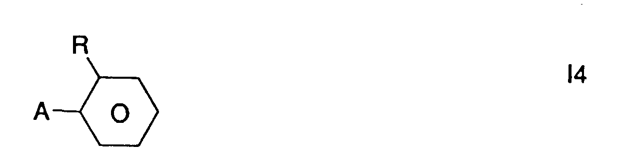

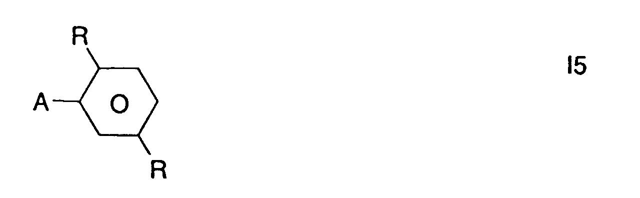

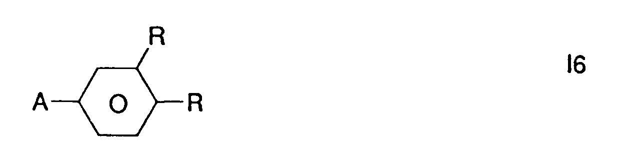

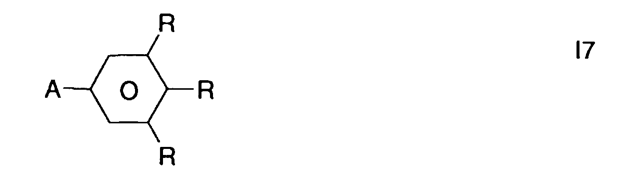

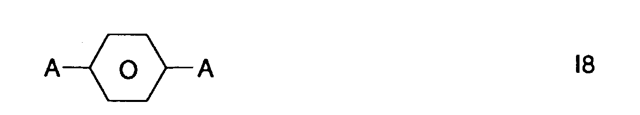

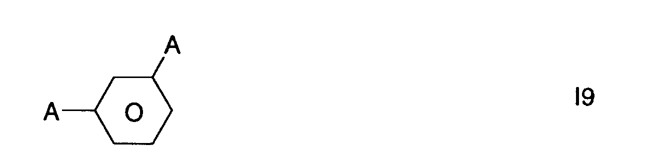

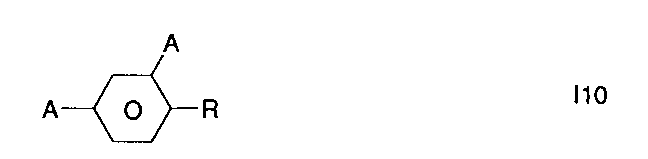

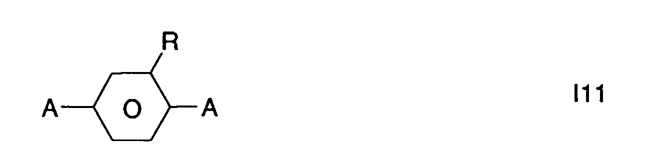

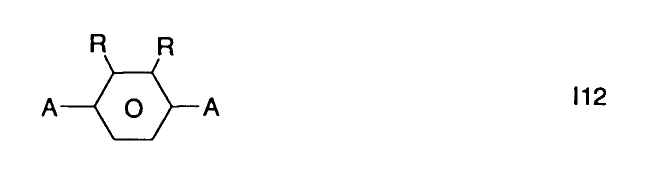

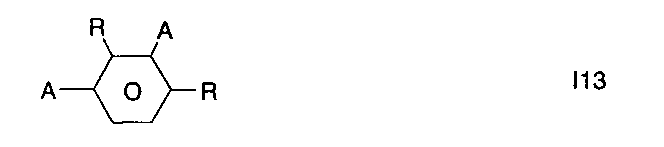

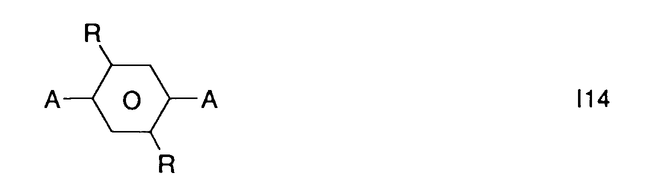

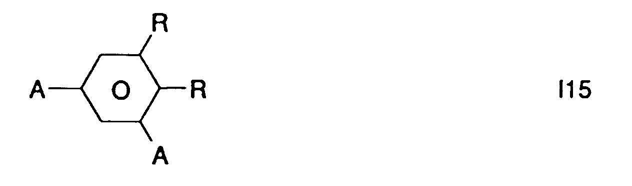

Darunter sind die Verbindungen der Formeln I1, I2, I5, I6, I7, I8 und I11 besonders bevorzugt.

Man erhält die Verbindungen der Formel I z.B. dadurch, daß man ein Benzaldehyd-Derivat der Formel II,

Phe-(CHO)ₙ II

worin Phe und n die angegebene Bedeutung besitzen, in Gegenwart einer Base oder einer Lewis-Säure mit Norcampher umsetzt.

Die Reaktion wird, in der Regel, in einem inerten Verdünnungsmittel, vorzugsweise einem protischen Lösungsmittel, insbesondere einem Alkohol, wie z.B. Methanol, Ethanol, Isopropanol oder tert. Butanol oder einem aprotischen Lösungsmittel, wie Diethylether, Toluol oder Cyclohexan oder Gemischen der genannten Lösungsmittel durchgeführt. Als Base setzt man vorzugsweise Alkalialkoholate, wie z.B. Natrium-Methylat, Natrium-Ethylat oder Kalium-tert.-Butylat ein. Als Lewis-Säure setzt man vorzugsweise Boranverbindungen ein.

Die Reaktion kann bei Temperaturen zwischen 0 °C und dem Siedepunkt des Reaktionsgemisches durchgeführt werden, vorzugsweise arbeitet man bei 25 bis 60 °C.

Im Falle von n = 1 setzt man in der Regel 0,8 bis 1,2 Mol Norcampher bezogen auf 1 Mol Verbindung der Formel II ein.

Im Falle n = 2 setzt man in der Regel 1,6 bis 2,4 Mol Norcampher bezogen auf 1 Mol Verbindung der Formel II ein.

Die Aldehyde der Formel II sind bekannt oder werden nach bekannten Methoden hergestellt.

Norcampher ist bekannt und käuflich erhältlich.

Gegenstand der Erfindung ist auch das Verfahren zur Herstellung der neuen Verbindungen der Formel I.

Ein weiterer Gegenstand der Erfindung ist eine kosmetische Zubereitung, welche in einem kosmetisch verträglichen Träger eine wirksame Menge mindestens eines Derivates der obigen Formel I enthält.

Das erfindungsgemäße kosmetische Mittel kann als Mittel zum Schutz der menschlichen Epidermis oder der Haare oder als Sonnenschutzmittel verwendet werden.

Gegenstand der Erfindung ist ferner ein Verfahren zum Schutz der Haut und natürlich oder sensibilisierter Haare von Sonnenstrahlen, wobei auf die Haut oder die Haare eine wirksame Menge mindestens einer Verbindung der Formel I aufgetragen wird.

Mit "sensibilisierten Haaren" sind Haare gemeint, welche einer Dauerwellenbehandlung, einem Färbe- oder Entfärbeprozeß unterzogen worden sind.

Gegenstand der Erfindung ist ferner eine gefärbte oder ungefärbte lichtstabilisierte kosmetische Zubereitung, welche eine wirksame Menge mindestens eines Benzyliden-Norcampher-Derivates der obigen Formel I umfaßt.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch, in Form ölig-alkoholischer, ölig-wäßriger oder wäßrig-alkoholischer Gele oder als feste Stifte vorliegen oder als Aerosol konfektioniert sein.

Es kann kosmetische Adjuvanzien enthalten, welche in diese Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel I ist in der Regel in einer Menge von 0,5 bis 10 %, vorzugsweise 1 bis 8 %, insbesondere 1 bis 5 %, bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Olen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine Verbindung der Formel I enthalten und andere UVB- und/ oder UVA-Filter umfassen können.

In diesem Fall beträgt die Menge des Filters der Formel I in der Regel zwischen 1,0 und 8,0 Gew.% bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor UV-Strahlen schützen, so kann es als Shampoo, Lotion, Gel oder Emulsion zum Ausspülen vorliegen, wobei die jeweilige Formulierung vor oder nach dem Shampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungsgemäßen Verbindung verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 1,0 bis 5,0 Gew.% der Verbindung der Formel I.

Die vorliegende Erfindung befaßt sich auch mit kosmetischen Mitteln, die mindestens eine Verbindung der Formel I als Mittel zum Schutz vor UV-Strahlen und als Antioxidationsmittel enthalten; diese Mittel umfassen Haarproduke, wie Haarlacke, Wasserwell-Lotionen zum Einlegen der Haare, gegebenenfalls zum Behandeln oder leichteren Frisieren, Shampoos, Färbeshampoos, Haarfärbemittel, Schminkprodukte, wie Nagellack, Cremes und Öle zur Hautbehandlung, Make-up (fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagems aufwerfen können. Derartige Mittel enthalten in der Regel 1,0 bis 5,0 Gew.% einer Verbindung der Formel I.

Ferner befaßt sich die Erfindung mit einem Verfahren zum Schutz der kosmetischen Mittel vor UV-Strahlen und Oxidation, wobei diesen Mitteln eine wirksame Menge mindestens einer Verbindung der Formel I zugesetzt wird.

Ein weiterer Gegenstand der Erfindung ist die Verwendung der Verbindungen der Formel I als Sonnenfilter von großer Absorptionsbreite in einem Wellenlängenbereich von 320 bis 400 nm.

Ein weiterer Gegenstand der Erfindung ist die Verwendung der Verbindungen der Formel I als kosmetische Produkte.

Wie oben bereits erwähnt, hat die Anmelderin im Laufe ihrer Untersuchungen außerdem festgestellt, daß die Verbindungen der Formel I eine signifikante pharmakologische Aktivität auf dem Gebiet der Präventivbehandlung von Entzündungen und Hautallergien zeigen.

Gegenstand der Erfindung sind auch die Verbindungen der Formel I zur Verwendung als Medikament.

Gegenstand der Erfindung ist ferner ein pharmazeutisches Mittel, welches eine wirksame Menge mindestens einer Verbindung der Formel I als Wirkstoff in einem nicht-toxischen Träger oder Exzipienten enthält.

Das erfindungsgemäße pharmazeutische Mittel kann oral oder topisch verabreicht werden.

Für die orale Verabreichung liegt das pharmazeutische Mittel in Form von Pastillen, Gelatinekapseln, Dragees, als Sirup, Suspension, Lösung, Emulsion etc. vor. Zur topischen Verabreichung liegt es als Salbe, Creme, Pomade, Lösung, Lotion, Gel, Spray, Suspension etc. vor.

Dieses Mittel kann inerte oder pharmakodynamisch aktive Additive enthalten, insbesondere Hydratisierungsmittel, Antibiotika, steroide oder nichtsteroide antiinflammatorische Mittel, Carotinoide und Mittel gegen Psoriasis.

Dieses Mittel kann auch Geschmacksverbesserungsmittel, Konservierungsmittel, Stabilisatoren, Feuchtigkeitsregulatoren, pH-Regulatoren, Modifikatoren für den osmotischen Druck, Emulgatoren, Lokalanästhetika, Puffer etc. enthalten.

Es kann außerdem in an sich bekannter Weise in Retard-Form oder in einer Form konditioniert sein, in welcher der Wirkstoff rasch freigesetzt wird.

Auch ohne weitere Ausführungen wird davon ausgegangen, daß ein Fachmann die obige Beschreibung in weitestem Umfang nutzen kann. Die bevorzugten Ausführungsformen sind deswegen lediglich als beschreibende, keineswegs als in irgendeine Weise limitierte Offenbarung aufzufassen.

Die vollständige Offenbarung aller vor- und nachstehend aufgeführten Anmeldungen, Patente und Veröffentlichungen sind durch Bezugnahme in diese Anmeldung eingeführt.

Die nachfolgenden Beispiele sind repräsentativ für die vorliegende Erfindung.

### Beispiel 1

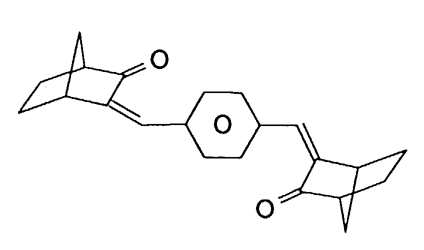

Eine Suspension aus 80 mMol (8,8 g) Norcampher und 120 mMol Natrium-Methylat (21,5 g einer 30%igen Lösung) in 100 ml Toluol werden 30' bei 50 °C gerührt. Anschließend werden 50 mMol (6,7 g) Terephthaldialdehyd eingetropft und die Mischung 1 h zum Rückfluß erhitzt. Danach wird auf Raumtemperatur abgekühlt und 175 ml Wasser zugegeben. Die Phasen werden getrennt und die wäßrige Phase mit Essigester extrahiert. Die vereinten organischen Extrakte werden mit 50 ml 1 N HCI-Lösung ausgeschüttelt, mit Wasser neutral gewaschen, getrocknet, filtriert und einrotiert.

Chromatographie mit Toluol/Ethylacetat 98:2 ergaben ein Rohprodukt, das zur weiteren Reinigung aus Isopropanol umkristallisiert wurde.
7,9 g = 62 %

| Elementaranalyse: | | | |
|---|---|---|---|
| berechnet: | C: 83,01 | H: 6,92 | O: 10,07 |
| gefunden: | C: 83,05 | H: 6,94 | O: 10,01 |

UV (Ethanol, c = 1 mg/100 ml): λₘₐₓ = 350 nm

Die Spektren entsprechen der erwarteten Verbindung.

### Beispiel 2

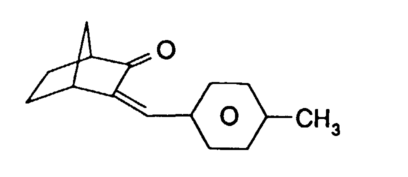

Eine Suspension aus 40 mMol (4,4 g) Norcampher und 60 mMol Natrium-Methylat in 50 ml Toluol wird 30' bei 50 °C gerührt. Anschließend werden 50 mMol (6,0 g) 4-Methylbenzaldehyd eingetropft und die Mischung zum Rückfluß erhitzt. Nach üblicher Aufarbeitung und Chromatographie erhält man 5,85 g = 69 % des Produkts.

| Elementaranalyse: | | | |
|---|---|---|---|
| berechnet: | C: 84,90 | H: 7,55 | O: 7,55 |
| gefunden: | C: 84,94 | H: 7,57 | O: 7,49 |

UV (Ethanol, c = 1 mg/100 ml): λₘₐₓ = 294 nm, E = 1,05

Analog wird hergestellt

### Beispiel 3

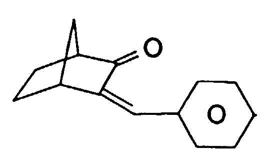

UV (Ethanol, c = 1 mg/100 ml): λₘₐₓ = 290 nm, E = 1,12

### Beispiel 4

### Sonnenschutzcreme (W/O)

| | | | % |
|---|---|---|---|
| A | Verbindung von Beispiel 1 | (1) | 3,00 |
| | Arlacel 581 | (2) | 7,00 |
| | Paraffinöl dünnflüssig (Art.-Nr. 7174) | (1) | 6,00 |
| | Arlamol S 7 | (2) | 2,00 |
| | Lunacera M | (3) | 5,00 |
| | Dow Corning 344 | (4) | 4,00 |
| | Miglyol 812 | (5) | 2,00 |
| | Oxynex 2004 (Art.-Nr. 6940) | (1) | 0,05 |
| B | Glycerin (Art.-Nr. 4093) | (1) | 2,00 |
| | Magnesium-Heptahydrat (Art.-Nr. 5882) | (1) | 0,17 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) ICI, Essen
(3) LW Fuller, Lüneburg
(4) Dow Corning, Düsseldorf
(5) Hüls Troisdorf AG, Witten

### Beispiel 5

### Sonnenschutzcreme (O/W)

| | | | % |
|---|---|---|---|
| A | Verbindung von Beispiel 2 | (1) | 3,00 |
| | Emulgator E 2155 | (2) | 8,00 |
| | Stearinsäure (Art.-Nr.671) | (1) | 2,00 |
| | Paraffinöl flüssig (Art.-Nr. 7162) | (1) | 6,00 |
| | Paraffin schüttfähig (Art.-Nr. 7158) | (1) | 6,00 |
| | Cetylalkohol (Art.-Nr. 989) | (1) | 2,50 |
| | Miglyol 812 | (3) | 9,50 |
| | Abil AV 200 | (2) | 0,50 |
| | Cetylpalmitat (Art.-Nr. 15419) | (1) | 5,50 |
| | (Tocopherolacetat (Art.-Nr. 500952) | (1) | 0,05 |
| B | Glycerin (Art.-Nr. 4093) | (1) | 3,00 |
| | Propandiol-1,2 (Art.-Nr. 7478) | (1) | 2,00 |
| | Karion F flüssig (Art.-Nr. 2993) | (1) | 5,00 |
| | Allantoin (Art.-Nr. 1015) | (1) | 0,25 |
| | Triethanolamin (Art.-Nr. 8377) | (1) | 0,50 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Th. Goldschmidt, Essen
(3) Hüls Troisdorf AG, Witten

### Beispiel 6

### Sonnenschutzmilch (W/O)

| | | | % |
|---|---|---|---|
| A | Verbindung von Beispiel 3 | (1) | 3,00 |
| | Pionier L-15 | (2) | 19,00 |
| | Paraffinöl dickflüssig (Art.-Nr. 7160) | (1) | 15,00 |
| B | Glycerin (Art.-Nr. 4093) | (1) | 5,00 |
| | Magnesium-Heptahydrat (Art.-Nr. 5882) | (1) | 0,50 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Hansen & Rosenthal, Hamburg

### Beispiel 7

### Sonnenschutzmilch (O/W)

| | | | % |
|---|---|---|---|
| A | Verbindung von Beispiel 2 | (1) | 3,00 |
| | Eumulgin B 1 | (2) | 3,00 |
| | Cutina MD | (2) | 8,00 |
| | Miglyol 812 | (3) | 7,00 |
| B | Glycerin (Art.-Nr. 4093) | (1) | 5,00 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Henkel, Düsseldorf
(3) Hüls Troisdorf AG, Witten

### Beispiel 8

### Sonnenschutzöl

| | | | % |
|---|---|---|---|
| A | Verbindung von Beispiel 1 | (1) | 3,00 |
| | Arlatone T | (2) | 2,00 |
| | Miglyol 812 | (3) | 16,00 |
| | Cetiol B | (4) | 22,50 |
| | Isopropylmyristat | (4) | 7,50 |
| | Paraffinöl dünnflüssig (Art.-Nr. 4174) | (1) | 48,85 |
| | Oxynex 2004 (Art.-Nr. 6940) | (1) | 0,05 |
| B | Parfümöl | (5) | 0,10 |

### Herstellung:

Phase A unter Rühren auf 70 °C erhitzen bis alle Komponenten gelöst sind, kaltrühren und bei 40 °C Phase B zugeben.

### Bezugsquellen:

(1) E. Merck, Darmstadt
(2) ICI, Essen
(3) Hüls Troisdorf AG, Witten
(4) Henkel, Düsseldorf
(5) Haarmann & Reimer, Holzminden

## Patentansprüche

1. Verwendung von Benzyliden-Norcampher-Derivaten der Formel I, 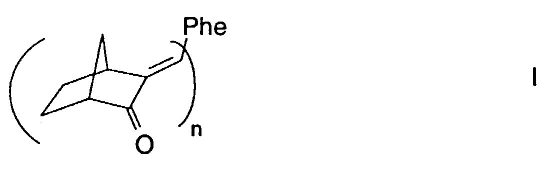 worin
Phe eine unsubstituierte oder durch 1 bis 5 Hydroxy-, Alkyl- oder Alkoxygruppen mit 1 bis 10 C-Atomen substituierte Phenylgruppe, und
n 1 oder 2
bedeuten,
als Lichtschutzfiltersubstanzen.

2. Benzyliden-Norcampher-Derivate der Formel I gemäß Anspruch 1, mit der Maßgabe daß Derivate der Formel, 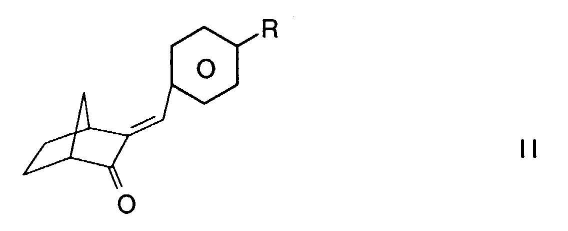 worin
R H oder OCH₃ bedeutet,
ausgenommen sind.

3. Benzyliden-Norcampher-Derivate der Formel I gemäß Anspruch 1, worin Phe eine durch 1 bis 2 Alkylgruppen mit 1 bis 10 C-Atomen substituierte Phenylgruppe bedeutet.

4. Verfahren zur Herstellung von Verbindungen der Formel I, dadurch gekennzeichnet, daß man ein Benzaldehyd-Derivat der Formel II,
Phe-(CHO)ₙ II
worin Phe und n die in Anspruch 1 angegebene Bedeutung haben, in Gegenwart einer Base oder einer Lewis-Säure mit Norcampher umsetzt.

5. Kosmetische Zubereitung, dadurch gekennzeichnet, daß sie eine wirksame Menge mindestens einer Verbindung der Formel I nach Anspruch 1 in einem kosmetisch verträglichen Träger enthält.

6. Kosmetische Zubereitung nach Anspruch 5, dadurch gekennzeichnet, daß sie zusätzlich einen UV-B-Filter enthält.

7. Verbindung der Formel I gemäß einem der Ansprüche 1 bis 3 zur Verwendung als Arzneimittel.

8. Pharmazeutische Zubereitung, dadurch gekennzeichnet, daß sie eine wirksame Menge mindestens einer Verbindung der Formel I nach einem der Ansprüche 1 bis 3 in einem physiologisch unbedenklichen Träger oder Exzipienten enthält.

9. Pharmazeutische Zubereitung nach Anspruch 12, dadurch gekennzeichnet, daß sie topisch angewandt wird.

## Claims

1. Use of benzylidenenorcamphor derivatives of the formula I 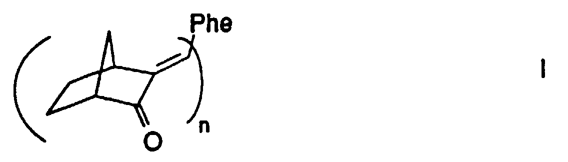 wherein
Phe is a phenyl group which is unsubstituted or substituted by 1 to 5 hydroxyl, alkyl or alkoxy groups having 1 to 10 C atoms, and
n is 1 or 2,
as sunscreen filter substances.

2. Benzylidenenorcamphor derivatives of the formula I according to Claim 1, with the proviso that derivatives of the formula 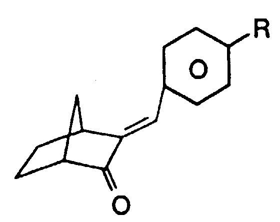 wherein
R is H, Cl or OCH₃,
are excluded.

3. Benzylidenenorcamphor derivatives of the formula I according to Claim 1, wherein Phe is a phenyl group which is substituted by 1 or 2 alkyl groups having 1 to 10 C atoms.

4. Process for the preparation of compounds of the formula I, characterized in that a benzaldehyde derivative of the formula II
Phe-(CHO)ₙ II
wherein Phe and n have the meaning indicated in Claim 1, is reacted with norcamphor in the presence of a base or of a Lewis acid.

5. Process according to Claim 4, characterized in that the base is an alkali metal alkoxide.

6. Cosmetic preparation, characterized in that it contains an effective amount of at least one compound of the formula I according to Claim 1 in a cosmetically tolerable carrier.

7. Cosmetic preparation according to Claim 6, characterized in that it contains 0.5 to 10% by weight of at least one compound of the formula I.

8. Cosmetic preparation according to one of Claims 6 or 7, characterized in that it additionally contains a UV-B filter.

9. Compound of the formula I according to one of Claims 1 to 3 for use as a medicament.

## Revendications

1. Utilisation de dérivés de benzylidène-norcamphre de formule I 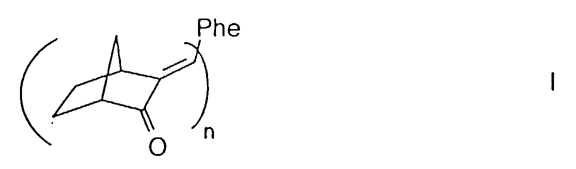 dans laquelle
Phe représente un groupe phényle non-substitué, ou substitué par 1 à 5 groupes hydroxy, alkyle ou alcoxy ayant de 1 à 10 atomes de carbone, et
n vaut 1 ou 2,
en tant que substances filtrantes assurant une protection contre la lumière.

2. Dérivés de benzylidène-norcamphre de formule I selon la revendication 1, à la condition que soient exclus les dérivés de formule 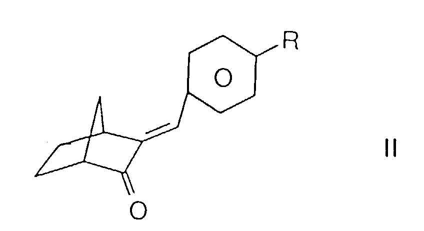 dans laquelle
R représente H ou OCH₃.

3. Dérivés de benzylidène-norcamphre de formule I selon la revendication 1, où Phe représente un groupe phényle substitué par 1 à 2 groupes alkyle ayant de 1 à 10 atomes de carbone.

4. Procédé de préparation de composés de formule I, caractérisé en ce qu'on fait réagir avec du norcamphre, en présence d'une base ou d'un acide de Lewis, un dérivé de benzaldéhyde de formule II
Phe - (CHO)ₙ II
dans laquelle Phe et n ont les significations données dans la revendication 1.

5. Préparation cosmétique, caractérisée en ce qu'elle contient une quantité efficace d'au moins un composé de formule I selon la revendication 1 dans un support compatible d'un point de vue cosmétique.

6. Préparation cosmétique selon la revendication 5, caractérisée en ce qu'elle contient en outre un filtre contre les rayons UV-B.

7. Composé de formule I selon l'une des revendications 1 à 3, pour utilisation en tant que médicament.

8. Préparation pharmaceutique, caractérisée en ce qu'elle contient une quantité efficace d'au moins un composé de formule I selon l'une des revendications 1 à 3 dans un support ou un excipient inoffensif du point de vue physiologique.

9. Préparation pharmaceutique selon la revendication 12, caractérisée en ce qu'elle est utilisée par voie topique.
